# EUROPEAN PATENT SPECIFICATION

(11) **EP 3 949 992 B1**
(45) Date of publication and mention of the grant of the patent: **25.11.2026**
(21) Application number: 20777051.2
(22) Date of filing: 26.03.2020
(51) Int. Cl.: A61K 31/713, A61P 31/20, A61P 35/00, A61K 38/00, C12N 15/113, G01N 33/50

(54) **YTHDC1 INHIBITOR AND ITS USE FOR THE TREATMENT OF HEPATITIS B**
YTHDC1 HEMMUNG UND BEHANDLUNG VON HEPATITIS B
INHIBITEUR DE YTHDC1 ET TRAITEMENT DE L'HÉPATITE B

(30) Priority: 26.03.2019 JP 2019058793
(43) Date of publication of application: 09.02.2022
(73) Proprietor: FUJIFILM Corporation, Tokyo 106-8620 (JP)
(72) Inventor: HABA Ryota, Toyama-shi, Toyama 930-8508 (JP); SHIMANE Kazuki, Toyama-shi, Toyama 930-8508 (JP); YAMASHITA Nozomi, Toyama-shi, Toyama 930-8508 (JP)
(74) Representative: Hoffmann Eitle
(86) International application number: PCT/JP2020/013625
(87) International publication number: WO 2020/196738

(56) References cited:
- WO-A1-2020/081937
- YANG JING ET AL: "Regulation of Virus Replication and T Cell Homeostasis by N6-Methyladenosine", VIROLOGICA SINICA, SPRINGER, DE, vol. 34, no. 1, 22 January 2019 (2019-01-22), pages 22 - 29, XP036728200, ISSN: 1674-0769, [retrieved on 20190122], DOI: 10.1007/S12250-018-0075-5
- ROUNDTREE IAN A ET AL: "YTHDC1 mediates nuclear export of N6-methyladenosine methylated mRNAs", vol. 6, 6 October 2017 (2017-10-06), pages 1 - 28, XP055950013, Retrieved from the Internet <URL:https://www.ncbi.nlm.nih.gov/pmc/articles/PMC5648532/pdf/elife-31311.pdf> DOI: 10.7554/eLife.31311
- PATIL DEEPAK P. ET AL: "m6A RNA methylation promotes XIST-mediated transcriptional repression", vol. 537, no. 7620, 1 September 2016 (2016-09-01), London, pages 369 - 373, XP055935472, ISSN: 0028-0836, Retrieved from the Internet <URL:https://www.nature.com/articles/nature19342.pdf> DOI: 10.1038/nature19342
- FENGCHUN YE ET AL: "Kaposi's Sarcoma-Associated Herpesvirus Utilizes and Manipulates RNA N 6 - Adenosine Methylation To Promote Lytic Replication", vol. 91, no. 16, 15 August 2017 (2017-08-15), US, pages e00466 - 17, XP055743141, ISSN: 0022-538X, Retrieved from the Internet <URL:https://www.ncbi.nlm.nih.gov/pmc/articles/PMC5533915/pdf/e00466-17.pdf> DOI: 10.1128/JVI.00466-17
- ZHANG, Y. ET AL.: "RNA-binding Protein YTHDF3 Suppresses Interferon-Dependent Antiviral Responses by Promoting FOX03 Translation.", PROC NATL ACAD SCI USA., vol. 116, no. 3, 15 January 2019 (2019-01-15), pages 976 - 981, XP055743130
- IMAM, H. ET AL.: "N6-methyladenosine modification of hepatitis B virus RNA differentially regulates the viral life cycle.", PROC NATL ACAD SCI USA., vol. 115, no. 35, 2018, pages 8829 - 8834, XP055743136
- YE, F. ET AL.: "Kaposi's Sarcoma-Associated Herpesvirus Utilizes and Manipulates RNA N 6- Adenosine Methylation To Promote Lytic Replication.", J VIROL., vol. 91, no. 16, 2017, pages 1 - 21, XP055743141
- BALACCO, DL ET AL.: "The m6A Writer: Rise of a Machine for Growing Tasks.", BIOCHEMISTRY, vol. 58, 5 February 2019 (2019-02-05), pages 363 - 378, XP055743144

## Description

### BACKGROUND OF THE INVENTION

### 1. Field of the Invention

The present invention relates to a pharmaceutical composition having an anti-hepatitis B virus activity and a method of screening a pharmaceutical drug candidate compound that inhibits the production of a hepatitis B virus protein.

### 2. Description of the Related Art

Hepatitis B virus (HBV) is a virus belonging to the genus Hepadnavirus and is a spherical DNA virus having a diameter of about 42 nm. The virus itself is a DNA virus consisting of a core having a diameter of 27 nm, which contains a coat (an envelope), an incomplete double-stranded DNA, a DNA polymerase, a reverse transcriptase, and the like, and is called a Dane particle. The Hepatitis B virus is formed in a double structure in which the outside consists of a hepatitis B surface (HBs) antigen (HBsAg) and the inside consists of a hepatitis B core antigen (HBcAg) and a genetic DNA (Non-Patent Document 1).

In addition to the Dane particle, the HBs antigen is present as a hollow particle, a small spherical particle, or a rod-shaped particle. The HBV genome DNA includes four kinds of open reading frames (ORFs), a pre-S/S gene region, a P gene region, an X gene region, and a pre-C/C gene region. The core promoter of the X gene region and the precore region of the C gene region are involved in the production of a hepatitis B envelope antigen (an HBe antigen, HBeAg) constituent protein, and the point mutation of this region makes the production of the HBe antigen constituent protein impossible, which results in the HBe antigen negativity.

In a case where HBV invades hepatocytes, the incomplete circular double-stranded DNA is incorporated into the nucleus of the hepatocytes and converted to a completely closed double-stranded DNA (a covalently closed circular DNA, cccDNA). In the state of chronically being infected by HBV, the cccDNA is present as a mini-chromosome at a quantity of 5 to 50 mini-chromosomes per hepatocyte. Four kinds of mRNAs are transcribed from the cccDNA in the hepatocyte nucleus, from which the HBs antigen, the HBc antigen, the HBe antigen, and the X protein, as structural proteins, and polymerases including a reverse transcriptase are translated. One of the mRNAs is incorporated into a core particle as the pregenomic RNA, a minus-strand DNA is synthesized by the action of the reverse transcriptase, and then a plus-strand DNA is synthesized to become an incomplete circular double-stranded DNA. Further, the incomplete circular double-stranded DNA is enveloped in an envelope formed of the HBs antigen to become a virus particle (a Dane particle), which is released into the blood. The hollow particle (the particle not having a nucleus with DNA) containing the HBs antigen, the HBc antigen, and the p22cr antigen, which are translated from the mRNAs, the HBe antigen that passes through the hepatocyte membrane are released and secreted in a large amount into the blood separately from the Dane particle blood release route. This action is conceived to be an action of escaping the attack of the host immune system against the HBV infection. The HBe antigen, the HBc antigen, or the p22cr antigen can be measured as a hepatitis B core-related (HBcr) antigen (HBcrAg) and is used as a marker of virus replication.

Hepatitis B is a kind of viral hepatitis caused by infection with HBV, and the number of infected people is said to be about 240 million worldwide. As described above, in a case of being infected with HBV, Dane particles are released into the blood, and a large amount of the viral protein is secreted into the blood separately from the release route of the Dane particles. It has been pointed out that among viral proteins, the HBs antigen, in particular, may interfere with the elimination of infected cells by the host immune system (Non-Patent Documents 2 and 3). Accordingly, in the medical treatment of hepatitis B, it is conceived important to reduce Dane particles, that is, to suppress the proliferation of the virus and to reduce viral proteins such as the HBs antigen.

Nucleic acid analogs such as entecavir and tenofovir are used as the first-choice drugs for hepatitis B. A nucleic acid analog pharmaceutical preparation can reduce the number of Dane particles in the blood, that is, suppress the proliferation of the virus, by inhibiting the activity of the HBV polymerase protein and inhibiting the generation of the incomplete circular double-stranded DNA. However, since the nucleic acid analog pharmaceutical preparation cannot reduce the HBs antigen, it is difficult to achieve the elimination of infected cells by the host immune system. For this reason, it has been desired to develop a pharmaceutical drug capable of achieving the reduction of the HBs antigen.

The YT-521B homology (YTH) domain-containing protein family is a protein group composed of YTHDC1, YTHDC2, YTHDF1, YTHDF2, and YTHDF3. It has been revealed that all of the above proteins bind to RNA through a recognition sequence containing a methylated adenosine (m6A) on the RNA and regulate biological phenomena such as RNA splicing and protein translation (Non-Patent Document 4). Focusing on the functions of YTH family molecules, it has been disclosed that compounds that inhibit the functions of YTH family molecules are useful for the medical treatment of infectious diseases and cancers (Patent Documents 1 and 2). In recent years, it has been reported that m6A is also present in HBV RNA, and it has been revealed that YTHDF2 and YTHDF3 bind to the HBV RNA and regulate the amount of an HBs antigen and an HBc antigen (Non-Patent Document 5). However, the data, disclosed in Non-Patent Document 5 show that the inhibition of YTHDF2 and YTHDF3 increases the HBs antigen and the HBc antigen, which are not data that show the anti-HBV activity.

### Prior Art Documents

### Patent Documents

Patent Document 1: JP2018-503663A
Patent Document 2: WO2010/134939A

### Non-Patent Documents

Non-Patent Document 1: Tanaka et al., Modern Media, Vol. 54, No. 12, pp. 347 to 352
Non-Patent Document 2: Brouw et al., Immunology, Vol. 126, pp. 280 to 289, 2008
Non-Patent Document 3: Vanlandschoot et al., Journal of General Virology, Vol. 83, pp. 1281 to 1289, 2002
Non-Patent Document 4: Liao et al., (2018) Genomics Proteomics Bioinformatics, Vol. 16, pp. 99 to 107
Non-Patent Document 5: Imam et al., (2018) Proc Natl Acad Sci USA, Vol. 115, No. 35, pp. 8829 to 8834 Non-patent Document 6: ZHANG, Y. et al., (2019) Proc Natl Acad Sci USA., Vol. 116, No. 3, pp 976 to 981

### SUMMARY OF THE INVENTION

An object of the present invention is to provide a novel pharmaceutical composition having an anti-hepatitis B virus (HBV) activity. In addition, another object of the present invention is to provide a pharmaceutical composition that inhibits the production of a hepatitis B virus protein and a pharmaceutical composition for treating hepatitis B. Further, another object of the present invention is to provide a method of screening a pharmaceutical drug candidate compound that inhibits the production of a hepatitis B virus protein.

As a result of diligent research on the above-described objects, the inventors of the present invention have found that a pharmaceutical composition that inhibits the expression of YTHDC1 has an ability to inhibit the production of an HBV protein and have completed the present invention.

That is, the present invention provides the followings. <1> A pharmaceutical composition for use in a method of treating hepatitis B in a subject, the pharmaceutical composition comprising an inhibitor to an expression or a function of a protein belonging to a YTHDC family, wherein the protein belonging to a YTHDC family is YTHDC1.
<2> The pharmaceutical composition for use according to <1>, wherein the pharmaceutical composition inhibits a production of a hepatitis B virus protein (an HBV protein), optionally wherein the HBV protein is an HBs antigen (HBsAg) protein.
<3> The pharmaceutical composition for use according to <1> or <2>, in which the pharmaceutical composition reduces an expression level of the HBV DNA.
<4> The pharmaceutical composition for use according to any one of <1> to <3>, in which the pharmaceutical composition reduces an expression level of a completely closed double-stranded DNA (a cccDNA).
<5> The pharmaceutical composition for use according to <1>, in which the inhibitor to the expression or the function of the protein belonging to the YTHDC family is selected from the group consisting of an antibody, a peptide, a protein, a non-peptide compound, a synthetic compound, an antisense nucleic acid, a double-stranded RNA, and a CRISPR-Cas vector system, each of which has an effective amount.
<6> The pharmaceutical composition for use according to <5>, in which the double-stranded RNA is an RNA interference agent.
<7> An in vitro method of screening a substance that inhibits a production of an HBV protein, the method comprising;
   (a) a step of bringing cells infected with HBV or cells expressing HBV into contact with a test substance that reduces an expression level of YTHDC1 or a test substance that inhibits a function or an activity of YTHDC1,
   (b) a step of measuring an ability of producing an HBV protein in the cells infected with HBV or the cells expressing HBV, and
   (c) a step of selecting a substance having an activity of inhibiting a production of the HBV protein of the step (b).
<8> The method according to <7>, in which the test substance of the step (a) is an antibody, a peptide, a protein, a non-peptide compound, a synthetic compound, an antisense nucleic acid, a double-stranded RNA, an adeno-associated virus vector, or a CRISPR-Cas vector system.
<9> The method according to <7>, in which the (b) includes a step of measuring an HBV protein in a culture supernatant of the cells infected with HBV or the cells expressing HBV.
<10> The method according to <7>, in which the substance of the step (c) is an antibody, a peptide, a protein, a non-peptide compound, a synthetic compound, an antisense nucleic acid, a double-stranded RNA, an adeno-associated virus vector, or a CRISPR-Cas vector system.

Further, the present invention provides the followings.
<a> Use of the pharmaceutical composition in a treatment method comprising administering the pharmaceutical composition according to any one of <1> to <6> to a subject who is infected with HBV or has a disease associated with HBV infection.
<b> The treatment method according to <a>, in which the disease associated with HBV infection is hepatitis B, liver cirrhosis caused by hepatitis B, or liver cancer caused by hepatitis B or liver cirrhosis.
<c> A kit for treating a disease associated with HBV infection, the kit comprising;
   the pharmaceutical composition according to any one of <1> to <6>, and
   an instruction in which a treatment method for a disease associated with HBV infection is described.
<d> The kit for treating a disease associated with HBV infection according to <c>, in which the treatment method for a disease associated with HBV infection includes administering the pharmaceutical composition to a subject who is infected with HBV or has a disease associated with HBV infection.
<e> A substance that inhibits an expression or a function of YTHDC1 the substance being for use in a treatment of inhibiting a production of a hepatitis B virus protein (an HBV protein).
<f> An inhibitor of a YTHDC1 for use in a treatment of hepatitis B.
<g> Use of a substance that inhibits an expression or a function of YTHDC1, the use being for producing a pharmaceutical composition that inhibits a production of a hepatitis B virus protein (an HBV protein).
<h> Use of an inhibitor of a YTHDC1, the use being for producing a pharmaceutical composition for treating hepatitis B in a subject.

The pharmaceutical composition according to the aspect of the present invention has an excellent anti-HBV activity and is useful as an anti-HBV agent. In addition, the screening method according to the aspect of the present invention is useful for screening a substance that inhibits the production of an HBV protein.

### BRIEF DESCRIPTION OF THE DRAWINGS

Fig. 1 shows the relative value (%) of cell viability in the dsRNA1-added well and the dsRNA2-added well in each concentration to the negative control dsRNA-added well.
Fig. 2 shows the relative value (%) of an HBs antigen (HBsAg) in the dsRNA1-added well and the dsRNA2-added well in each concentration to the negative control dsRNA-added well.
Fig. 3 shows the relative value (%) of the DNA of HBV (HBV DNA) in the dsRNA1-added well and the dsRNA2-added well in each concentration to the negative control dsRNA-added well.
Fig. 4 shows the relative value (%) of the YTHDC1 expression level in the dsRNA1-added well and the dsRNA2-added well to the YTHDC1 expression level in the negative control dsRNA-added well.
Fig. 5 shows the relative value (%) of cell viability in the dsRNA3-added well and the dsRNA4-added well in each concentration to the negative control dsRNA-added well.
Fig. 6 shows the relative value (%) of an HBs antigen (HBsAg) in the dsRNA3-added well and the dsRNA4-added well in each concentration to the negative control dsRNA-added well.
Fig. 7 shows the relative value (%) of the DNA of HBV (HBV DNA) in the dsRNA3-added well and the dsRNA4-added well in each concentration to the negative control dsRNA-added well.
Fig. 8 shows the relative value (%) of the YTHDC1 expression level in the dsRNA3-added well and the dsRNA4-added well to the YTHDC1 expression level in the negative control dsRNA-added well.

### DESCRIPTION OF THE PREFERRED EMBODIMENTS

Hereinafter, the present invention will be described in detail.

In the present specification, unless otherwise specified, each term has the following meaning.

The prevention means the inhibition of the onset of a disease, the reduction of the risk of the onset of a disease, or the delay of the onset of a disease.

The medical treatment means the amelioration or suppression of progression of a disease or a condition.

The treatment means the prevention or medical treatment of various diseases.

The effective amount means a therapeutically effective amount, or a preventive effective amount.

Therapeutically effective amount is an amount sufficient to stabilize HBV infection, reduce HBV infection, or eradicate HBV infection in an infected subject. In addition, the preventive effective amount is an amount sufficient for preventing HBV infection in a subject under the risk of infection.

The subject means a mammal including a human.

### <Hepatitis B virus (HBV)>

Hepatitis B virus (HBV) means a virus that has the ability to develop hepatitis B. HBV is currently classified into eight genotypes, from A type to H type, based on the difference in the base sequence, which is derived from the gene mutation. The HBV to be prevented or medically treated with the pharmaceutical composition according to the embodiment of the present invention includes all genotypes thereof.

In the present invention, as a disease associated with HBV infection, hepatitis B is mentioned, and examples thereof include acute hepatitis, chronic hepatitis, and fulminant hepatitis. In addition, liver cirrhosis, liver fibrosis, and liver cancer such as hepatocellular carcinoma are also included in a case where the disease is caused by HBV infection to a living body including a human.

### <Hepatitis B virus protein (HBV protein)>

The hepatitis B virus protein (the HBV protein) is a protein constituting HBV, and examples thereof include an HBs antigen, an HBc antigen, and an HBe antigen.

In the present invention, the HBV protein is not particularly limited; however, it is preferably an HBs antigen.

### <Pharmaceutical composition>

The pharmaceutical composition according to the embodiment of the present invention is a pharmaceutical composition containing a "substance", specifically a "substance that inhibits the production of an HBV protein", and a pharmaceutically acceptable carrier.

The pharmaceutical composition according to the embodiment of the present invention contains, as a substance that inhibits the production of an HBV protein, a substance that inhibits the expression or the function of YTHDC1.

The pharmaceutical composition according to the embodiment of the present invention preferably has a substance that inhibits the production of an HBV protein and more preferably a substance that inhibits the production of the HBs antigen.

Examples of the "substance" include an antibody, a peptide, a protein, a non-peptide compound, a synthetic compound, an antisense nucleic acid, a double-stranded RNA, an adeno-associated virus vector, and a CRISPR-Cas vector system.

The "antisense nucleic acid" is a nucleic acid including a base sequence or a part thereof, which is complementary or substantially complementary to a base sequence of a "sense" nucleic acid that encodes a protein, such as a coding strand of a double-stranded cDNA molecule, an mRNA sequence, or a coding strand of a gene, and binds to a target base sequence to form a specific and stable double strand, and thus has the function of inhibiting protein synthesis.

A "double-stranded RNA" includes, but is not limited to, a nucleic acid that interferes with or inhibits the expression of a target gene by a short interfering RNA (a siRNA), a short hairpin RNA (an shRNA), or RNA interference (RNAi).

An "RNA interference agent" is a nucleic acid that interferes with or inhibits the expression of a target gene through RNA interference.

The RNA interference is a post-transcriptional target gene silencing technique that uses a double-stranded RNA (a dsRNA) to degrade an mRNA including the same sequence as the dsRNA (Sharp et al., Science, 287, 2431 to 2432, 2000); Zamore et al., Cell, Vol. 101, pp. 25 to 33, 2000; Tuschl et al., Genes & Development, Vol. 13, 3191 to 3197, 1999). The RNA interference occurs in a case where an endogenous ribonuclease cleaves a long dsRNA to generate a shorter RNA having a length of 21 or 22 nucleotides, called a small interfering RNA (siRNA). This siRNA mediates the degradation of a target mRNA. Synthesis kits for RNAi are commercially available, for example, from New England Biolabs and Ambion. In an aspect of the synthesis of RNAi, one or more of the above synthesis kit for use in antisense RNA can be used.

The substance contained in the pharmaceutical composition according to the embodiment of the present invention is preferably an antisense nucleic acid or a double-stranded RNA, and more preferably a double-stranded RNA.

Further, in the case of the double-stranded RNA, it is particularly preferably an RNA interference agent.

The "adeno-associated virus" is a single-stranded DNA virus, which belongs to the family Parvoviridae and consists of about 4,700 bases, has no envelope and has a capsid of a regular icosahedral structure having a diameter of 20 to 30 nm. Since the adeno-associated virus recognizes heparan sulfate proteoglycan which is a universal component of the cell membrane to infect a cell, the range of hosts thereof is wide.

Any gene or nucleic acid can be inserted into the genome of the adeno-associated virus. For this reason, it is possible to introduce a gene or nucleic acid of interest by infecting a target cell with a genetically modified adeno-associated virus, and thus it is possible to use the adeno-associated virus as a vector for gene transfer. Further, since the "adeno-associated virus vector" is non-pathogenic and enables the gene transfer into a terminally differentiated non-dividing cell, it is expected to be applied to gene therapy.

It is known that adeno-associated viruses have different infection directivity to tissues and cells depending on the difference in serotype (Ozawa et al., Drug Delivery System, Vol. 22, No. 6, pp. 643 to 650, 2007).

The substance contained in the pharmaceutical composition according to the embodiment of the present invention is preferably an adeno-associated virus type 5, an adeno-associated virus type 7, an adeno-associated virus type 8, or an adeno-associated virus type 9, and more preferably an adeno-associated virus type 8.

The "CRISPR-Cas system" is a system that uses, for genome editing, the clustered regulatory interspaced short palindromic repeats/CRISPR-associated protein (CRISPR/Cas) system discovered as acquired immunity of eubacteria and archaea (Jinek et al., Science, Vol. 337, pp. 816-821, 2012). Specifically, in the genomic region called the CRISPR region of the above bacteria, a plurality of cassettes in which a fragmented foreign DNA (20 bp) is incorporated are repeated, and a different foreign DNA is incorporated into each of the cassettes.

It is conceived that each of the cassettes is generated by fragmenting a DNA of a foreign introduced species (for example, a phage) and incorporating the fragmented DNA into the CRISPR region in a case where the DNA is incorporated into the cell.

Each of the cassettes encodes a CRISPR RNA (a crRNA). The crRNA has a protospacer sequence and a sequence complementary to the trans-crRNA (the tracrRNA) described later. The protospacer sequence has a length of 20 nucleotides and has a sequence complementary to the target DNA sequence. The protospacer sequence need not have the complementarity of 100% with the target sequence and is allowed to have mismatches in the region of 6 nucleotides from the 5' end. The crRNA forms a complex with the trans-crRNA (the tracrRNA), which is separately transcribed, through the complementary moiety. Further, the complex of the crRNA and the tracrRNA forms a complex with Cas9 endonuclease. The protospacer moiety in the crRNA forms a complementary hybrid with the foreign DNA, which leads the Cas9 endonuclease to the target sequence of the foreign DNA. The foreign DNA is cleaved by the Cas9 endonuclease at the site of the target sequence, and the CRISPR-Cas9 system protects the host from the foreign DNA.

As described above, the CRISPR-Cas9 system has been discovered as a system of eubacteria and archaea, which functions as acquired immunity to a new foreign DNA; however, the CRISPR-Cas9 system makes it possible to sequence-dependently cleave a mammalian genome by designing a protospacer consisting of a foreign DNA, whereby the mammalian genome can be edited.

The "CRISPR-Cas vector system" is a vector system containing a crRNA, a tracrRNA sequence, or a guide RNA (a gRNA) having both functions of the crRNA and the tracrRNA, and a Cas gene sequence. Currently, the predominantly used system is, but is not limited to, a second type CRISPR-Cas9 system, which is derived from Streptococcus pyogenes (S. pyogenes).

The pharmaceutically acceptable carrier includes an excipient, a diluent, a bulking agent, a disintegrating agent, a stabilizer, a preservative, a buffer, an emulsifier, a fragrance, a colorant, a sweetener, a thickening agent, a taste improving agent, a dissolution auxiliary agent, and other additives. In a case where one or more of such carriers are used, it is possible to prepare pharmaceutical compositions having forms such as a tablet, a capsule, a powdered drug, a syrup, a granule, a pill, a suspension, an emulsion, a powder preparation, a suppository, an eye drop, a nasal drop, an ear drop, a patch, an ointment, an injection agent, a liquid drug, a troche, and an elixir.

These pharmaceutical compositions can be administered orally or parenterally. Examples of other forms for parenteral administration include an external liquid drug that contains one or more active substances and is prescribed according to the rule and a suppository for enteric administration.

In addition, the dose and the frequency of administration of the pharmaceutical composition according to the embodiment of the present invention can be appropriately selected depending on the sex, weight, age, severity, symptom, and the like of the patient. In general, for adults, a daily dose of 0.01 to 1,000 mg/kg may be dividedly administered one to several times by oral or parenteral administration (for example, injection, drip infusion, or administration to the rectal region).

### <Protein belonging to YTHDC family>

The YT-521B homology (YTH) domain-containing protein family is a protein group composed of YTHDC1, YTHDC2, YTHDF1, YTHDF2, and YTHDF3. It has been revealed that all of the above proteins bind to RNA through a recognition sequence containing a methylated adenosine (m6A) on the RNA and regulate biological phenomena such as RNA splicing and protein translation (Liao et al., (2018) Genomics Proteomics Bioinformatics, Vol. 16, pp. 99 to 107).

In addition, examples of the protein belonging to the YTH domain containing (YTHDC) family include YTHDC1 and YTHDC2.

The pharmaceutical composition according to the embodiment of the present invention inhibits the expression or the function of YTHDC1.

The pharmaceutical composition according to the embodiment of the present invention is useful as a pharmaceutical composition for treating hepatitis B in a subject, where the pharmaceutical composition contains an inhibitor to the expression or the function of the protein belonging to the YTHDC family.

### <Screening method>

The screening method according to the embodiment of the present invention is useful as a method of screening a substance that inhibits the production of an HBV protein.

Examples of the screening method according to the embodiment of the present invention include a method including;
(a) a step of bringing cells infected with HBV or cells expressing HBV into contact with a test substance that reduces an expression level of a protein belonging to a YTHDC1 or a test substance that inhibits a function or an activity of the protein,
(b) a step of measuring an ability of producing an HBV protein in the cells infected with HBV or the cells expressing HBV, and
(c) a step of selecting a substance having an activity of inhibiting a production of the HBV protein of the step (b).

(a) A step of bringing cells infected with HBV or cells expressing HBV into contact with a test substance that reduces an expression level of YTHDC1 protein or a test substance that inhibits a function or an activity of the protein

Examples of HBV-infected cells include a cultured human primary hepatocyte infected with HBV clone C_JPNAT (GenBank: AB246345.1), and a HepG2 cell forcibly expressing a sodium taurocholate coating polypeptide (NTCP), which is infected with HBV clone C_JPNAT (GenBank: AB246345.1). The combination of the HBV clone and the cell described above is not particularly limited.

Examples of the cells expressing HBV include a HepG2.2.15 cell, and a HepG2 cell, a Huh-7 cell, a HepaRG cell, and a cultured human primary hepatocyte, which forcibly express a plasmid formed by containing a 1.24-fold lengthened genome sequence of HBV clone C_JPNAT (GenBank: AB246345.1). The above HBV clone is not particularly limited. The length of the sequence derived from the HBV genome, which is inserted into the plasmid formed by containing the genome sequence of the HBV clone is not particularly limited as long as it is at least 1.24 folds the length of the genome sequence of the HBV clone.

Examples of the test substance that reduces the expression level of a YTHDC1 protein or the test substance that inhibit the function or the activity of the protein include a substance selected from an antibody, a peptide, a protein, a non-peptide compound, a synthetic compound, an antisense nucleic acid, a double-stranded RNA, an adeno-associated virus vector, and a CRISPR-Cas vector system.

The test substance that is used in the screening method according to the embodiment of the present invention is preferably an antisense nucleic acid or a double-stranded RNA, and more preferably a double-stranded RNA.

(b) A step of measuring an ability of producing an HBV protein in the cells infected with HBV or the cells expressing HBV

Examples of the step of measuring the ability of producing HBV protein include a step of measuring an HBV protein in a culture supernatant of the cells infected with HBV or the cells expressing HBV.

The HBV protein can be measured using the collected culture supernatant, for example, by a chemiluminescence enzyme-linked immunosorbent assay (a CLEIA method) or an enzyme-linked immunosorbent assay (an ELISA method), or by a method similar thereto.

(c) A step of selecting a substance having an activity of inhibiting a production of the HBV protein of the step (b)

For example, in a case where test substances are allowed to act in the (b), it is possible to select a substance that inhibits the production of the HBV protein by 30% or more, preferably 50% or more, more preferably 70% or more, still more preferably 90% or more, as compared with the negative control.

### <Use application of pharmaceutical composition>

The pharmaceutical composition set out in the claims is useful in the medical treatment or prevention of HBV infection.

The pharmaceutical composition for use according to the claims can inhibit the production of an HBV protein, particularly the HBs antigen. In addition, the HBV gene expression (the HBV DNA production) can be inhibited. As a result, the pharmaceutical composition according to the claims is useful for the medical treatment or prevention of HBV infection.

The present invention relates to the use of the pharmaceutical composition according to the claims for inhibiting the production of the HBs antigen. The present invention relates to the use of the pharmaceutical composition according to the claims for inhibiting the DNA production of HBV. The present invention relates to the use of the pharmaceutical composition according to the claims for inhibiting the gene expression of HBV.

The present invention relates to the use of the pharmaceutical composition according to the claims for the medical treatment or prevention of HBV infection.

Next, the present invention will be described with reference to Test Examples; however, the present invention is not limited thereto.

### [Examples]

### [Test Example 1]

### (Anti-HBV assay)

HepG 2.2.15 cells obtained by introducing the HBV genome into a hepatoblastoma cell line HepG2 (Proc Natl Acad Sci USA, Vol. 84, pp. 1005 to 1009, 1987), which are cells continuously producing viruses, were used, and the anti-HBV activity obtained by the inhibition of YTHDC1 was evaluated by the following procedure.

The following medium was used as a cell culture medium.

DMEM/F-12, GlutaMAX (manufactured by Thermo Fisher Scientific, Inc.) + 5 µg/mL insulin (manufactured by FUJIFILM Wako Pure Chemical Corporation) + 1% penicillin/streptomycin (manufactured by Sigma-Aldrich Co., LLC) + 10 mmol/L HEPES (manufactured by Sigma-Aldrich Co., LLC) + 50 µmol/L hydrocortisone (manufactured by Sigma-Aldrich Co., LLC) +10 % FBS (manufactured by Equitech-Bio Inc.)

dsRNAs used for transfection have the following sequences (designated by SEQ ID NO: 1 to SEQ ID NO: 4) (manufactured by Thermo Fisher Scientific, Inc.).
dsRNA 1 is complementary to the first sequence of the human YTHDC1 gene. 5'-GGA AUU UCA UAA CAU GGG Att-3' (SEQ ID NO: 1) 5'-UCC CAU GUU AUG AAA UUC Cct-3' (SEQ ID NO: 2)
dsRNA 2 is complementary to the second sequence of the human YTHDC1 gene. 5'-CAG UAA AGA UCG GAC GUG Att-3' (SEQ ID NO: 3) 5'-UCA CGU CCG AUC UUU ACU Ggt-3' (SEQ ID NO: 4)

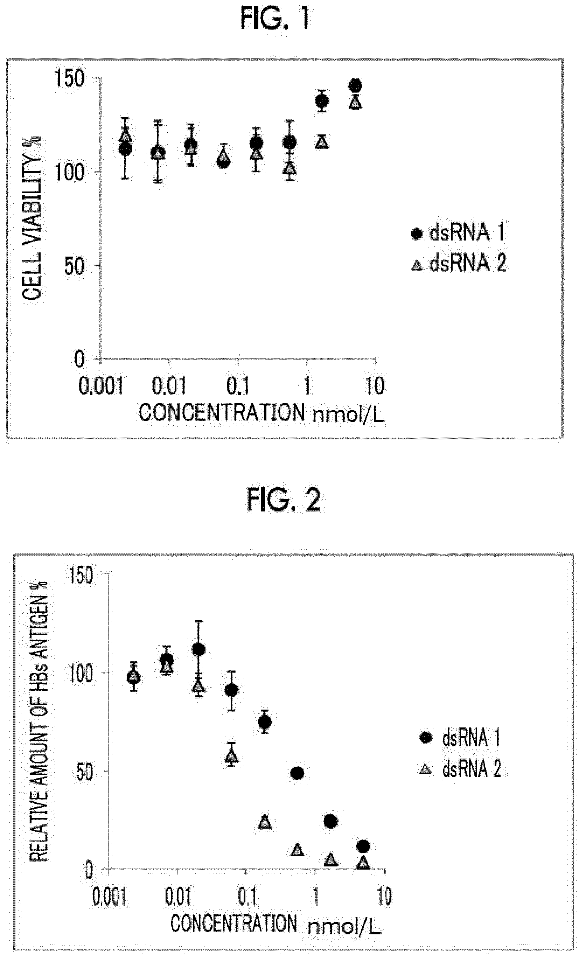

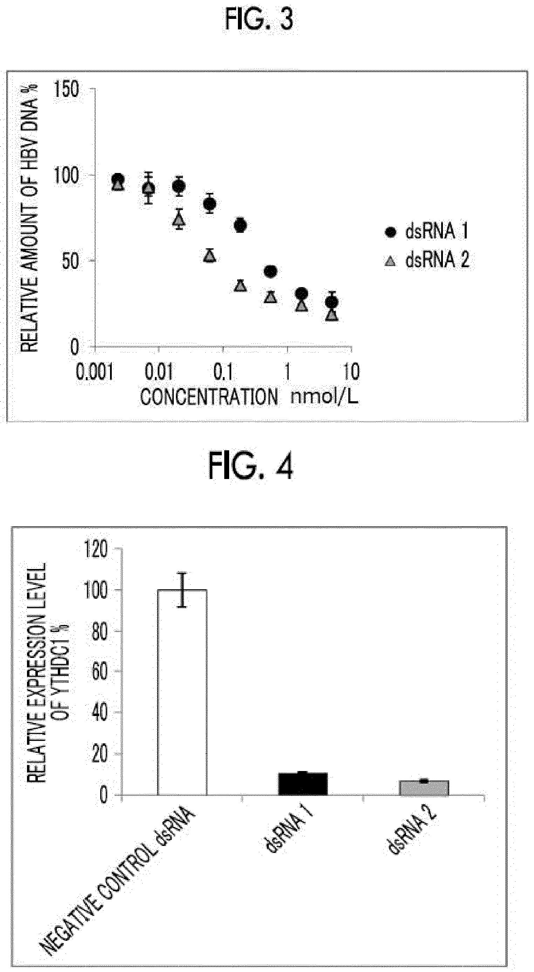

In addition, Negative Control No. 1 siRNA (manufactured by Thermo Fisher Scientific, Inc.) was used as a negative control dsRNA.
(1) HepG 2.2.15 cells were suspended in the above medium to prepare a HepG 2.2.15 cell suspension of 2 × 10⁵ cells/mL. The dsRNA was diluted with Nuclease-Free Water (not DEPC-Treated) (manufactured by Thermo Fisher Scientific, Inc.) to prepare dsRNA solutions of 3-fold dilution series from 0.229 nmol/L to 500 nmol/L. 1 µL of the prepared dsRNA solution and 0.3 µL of Lipofectamine RNAiMAX (manufactured by Thermo Fisher Scientific, Inc.) were diluted with 8.7 µL of serum-free medium (manufactured by Thermo Fisher Scientific, Inc.) and incubated at room temperature for 5 minutes. 10 µL of this dsRNA-Lipofectamine RNAiMAX mixture was diluted with 40 µL of a cell culture medium to adjust the volume to 50 µL. This diluted mixture was mixed with 50 µL of the above-described HepG 2.2.15 cell suspension, seeded on a 96-well microtiter plate, and incubated in a 5% CO₂ incubator at 37°C for 3 days. Next, the medium was exchanged at 100 µL/well with the above-described cell culture medium, and incubation was further carried out at 37°C for 3 days in a 5% CO₂ incubator. Then, the culture supernatant was collected, and the cell viability was measured using CellTiter96 AQueous One Solution Cell Proliferation Assay (manufactured by Promega Corporation) (Fig. 1). The amount of the HBs antigen in the culture supernatant was measured using AlphaLISA Hepatitis B Virus Surface Antigen (HBsAg) Kit (manufactured by PerkinElmer, Inc.). In addition, the collected culture supernatant was treated with a mixture of Buffer AL (manufactured by Qiagen) and Proteinase K (manufactured by Thermo Fisher Scientific, Inc.), and the DNA of HBV (the HBV DNA) in the obtained solution was quantified by a real-time PCR method. Regarding each of the markers, the relative value (%) in each of the dsRNA-added wells in each concentration to the negative control dsRNA-added well was calculated (Fig. 2 and Fig. 3).
(2) HepG 2.2.15 cells were suspended in the above medium to prepare a HepG 2.2.15 cell suspension of 2 × 10⁵ cells/mL. 10 µL of the dsRNA (500 nM) and 3 µL of Lipofectamine RNAiMAX (manufactured by Thermo Fisher Scientific, Inc.) were diluted with 87 µL of serum-free medium (manufactured by Thermo Fisher Scientific, Inc.) and incubated at room temperature for 5 minutes. 100 µL of this dsRNA-Lipofectamine RNAiMAX mixture was diluted with 400 µL of a cell culture medium to adjust the volume to 500 µL. This diluted mixture was mixed with 500 µL of the above-described HepG 2.2.15 cell suspension, seeded on a 12-well microtiter plate, and incubated in a 5% CO₂ incubator at 37°C for 3 days. Next, the medium was exchanged at 1,000 µL/well with the above-described cell culture medium, and incubation was further carried out at 37°C for 3 days in a 5% CO₂ incubator. Then, the culture supernatant was discarded, the cells were washed with phosphate buffered saline (1,000 µL/well, manufactured by FUJIFILM Wako Pure Chemical Corporation), and total RNA was extracted using an RNeasy Mini Kit (manufactured by Qiagen). 1 µg of the extracted total RNA was reverse transcribed using a PrimeScript RT Reagent Kit with gDNA Eraser (manufactured by Takara Bio Inc.) to prepare cDNA. YTHDC1 and glyceraldehyde-3-phosphate dehydrogenase (GAPDH) as a housekeeping gene, in the prepared cDNA, were quantified by a real-time PCR method, the YTHDC1 expression level corrected by GAPDH was calculated, and then the relative value (%) of the expression level of YTHDC1 in each of the dsRNA-added wells to the expression level of YTHDC1 in the negative control dsRNA-added well was calculated (Fig. 4).

It has been revealed that in a case where the expression level of YTHDC1 is reduced, the amount of the HBs antigen is reduced whereas the cell viability is not affected. It has also been revealed that the HBV DNA also decreased.

### [Test Example 2]

### (Anti-HBV assay using dsRNAs derived from different manufacturers)

Using HepG 2.2.15 cells, the anti-HBV activity due to the inhibition of YTHDC1 was evaluated by the following procedure.

The following medium was used as a cell culture medium.

DMEM/F-12, GlutaMAX (manufactured by Thermo Fisher Scientific, Inc.) + 5 µg/mL insulin (manufactured by FUJIFILM Wako Pure Chemical Corporation) + 1% penicillin/streptomycin (manufactured by FUJIFILM Wako Pure Chemical Corporation) + 10 mmol/L HEPES (manufactured by FUJIFILM Wako Pure Chemical Corporation) + 50 µmol/L hydrocortisone (manufactured by FUJIFILM Wako Pure Chemical Corporation) +10 % FBS (manufactured by Equitech-Bio Inc.)
dsRNAs used for transfection have the following sequences (designated by SEQ ID NO: 5 to SEQ ID NO: 8).
dsRNA 3 (manufactured by Horizon Discovery Group plc) is complementary to the third sequence of the human YTHDC1 gene.
5'-UCU AGA UGC UGA UCG GAA Att-3' (SEQ ID NO: 5)
5'-UUU CCG AUC AGC AUC UAG Att-3' (SEQ ID NO: 6)
dsRNA 4 (manufactured by Sigma-Aldrich Co., LLC) is complementary to the fourth sequence of the human YTHDC1 gene.
5'-GAA UGA ACA UAA ACC AGU Att-3' (SEQ ID NO: 7)
5'-UAC UGG UUU AUG UUC AUU Ctt-3' (SEQ ID NO: 8)

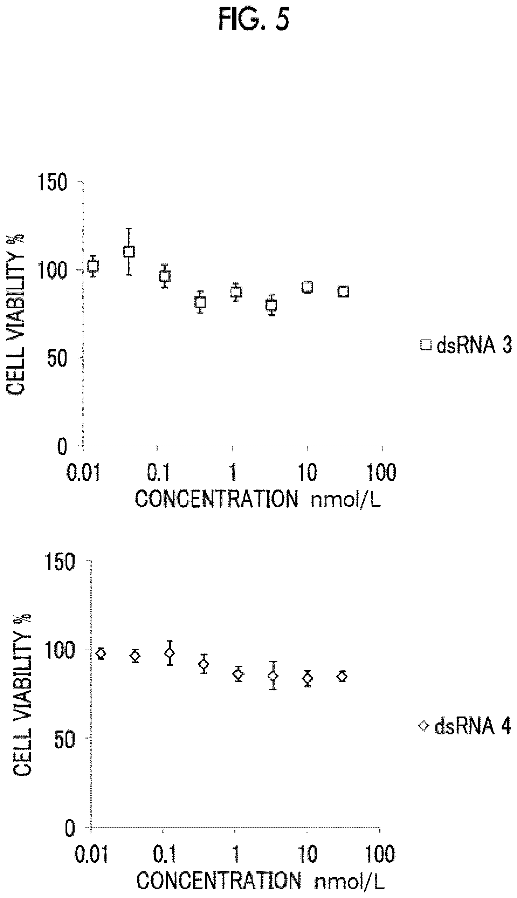

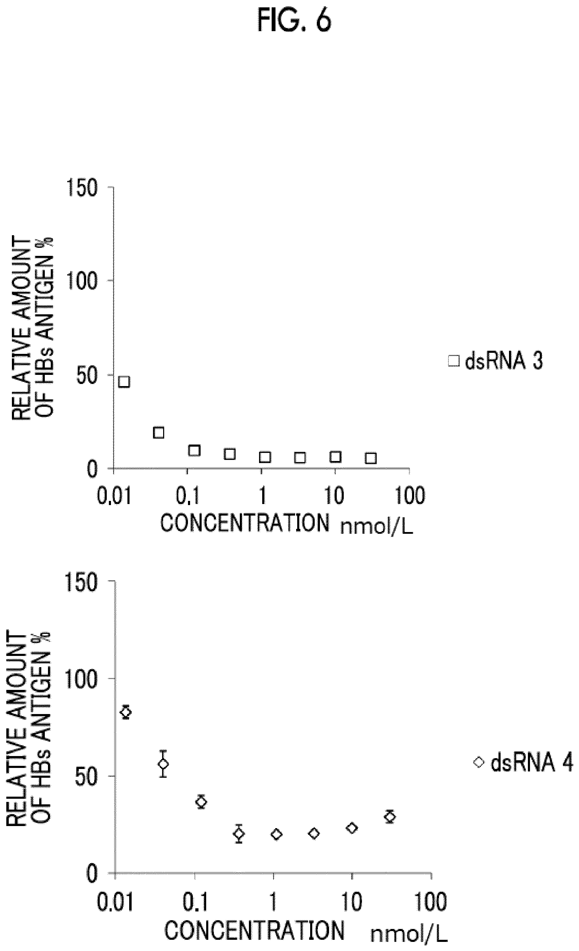

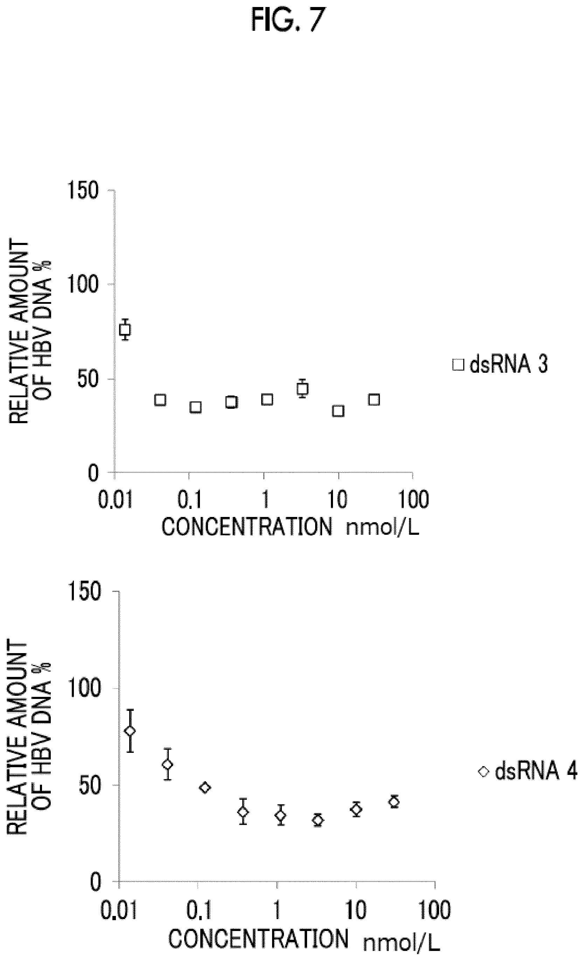

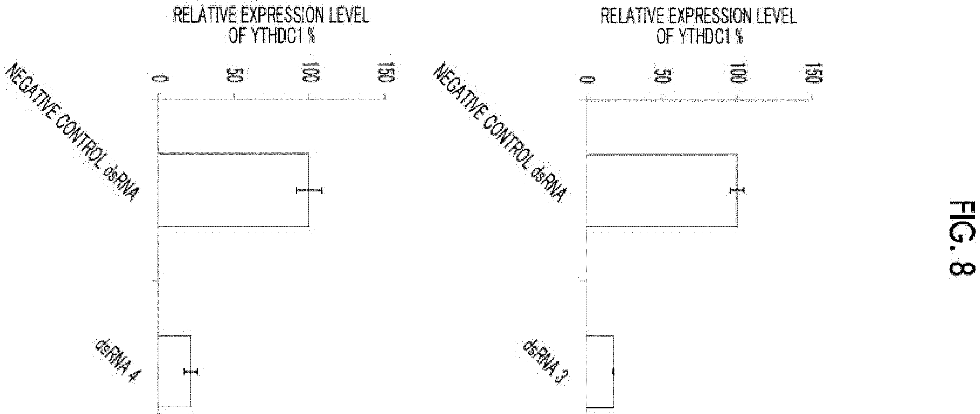

In addition, an On-TARGETplus Non-Targeting pool (manufactured by Horizon Discovery Group plc) or Mission_Negative control SIC-002 (manufactured by Sigma-Aldrich Co., LLC) was used as a negative control dsRNA.
(1) HepG 2.2.15 cells were suspended in the above medium to prepare a HepG 2.2.15 cell suspension of 2 × 10⁵ cells/mL. The dsRNA was diluted with Nuclease-Free Water (not DEPC-Treated) (manufactured by Thermo Fisher Scientific, Inc.) or 1 × siRNA Buffer (manufactured by Horizon Discovery Group plc) to prepare dsRNA solutions of 3-fold dilution series from 0.457 nmol/L to 1,000 nmol/L. 3 µL of the prepared dsRNA solution and 0.3 µL of Lipofectamine RNAiMAX (manufactured by Thermo Fisher Scientific, Inc.) were diluted with 6.7 µL of serum-free medium (manufactured by Thermo Fisher Scientific, Inc.) and incubated at room temperature for 5 minutes. 10 µL of this dsRNA-Lipofectamine RNAiMAX mixture was diluted with 40 µL of a cell culture medium to adjust the volume to 50 µL. This diluted mixture was mixed with 50 µL of the above-described HepG 2.2.15 cell suspension, seeded on a 96-well microtiter plate, and incubated in a 5% CO₂ incubator at 37°C for 3 days. Next, the medium was exchanged at 100 µL/well with the above-described cell culture medium, and incubation was further carried out at 37°C for 3 days in a 5% CO₂ incubator. Then, the culture supernatant was collected, and the cell viability was measured using CellTiter96 AQueous One Solution Cell Proliferation Assay (manufactured by Promega Corporation) (Fig. 5). The amount of the HBs antigen in the culture supernatant was measured using AlphaLISA Hepatitis B Virus Surface Antigen (HBsAg) Kit (manufactured by PerkinElmer, Inc.). In addition, the collected culture supernatant was treated with a mixture of Buffer AL (manufactured by Qiagen) and Proteinase K (manufactured by Thermo Fisher Scientific, Inc.), and the DNA of HBV (the HBV DNA) in the obtained solution was quantified by a real-time PCR method. Regarding each of the markers, the relative value (%) in each of the dsRNA-added wells in each concentration to the negative control dsRNA-added well was calculated (Fig. 6 and Fig. 7).
(2) HepG 2.2.15 cells were suspended in the above medium to prepare a HepG 2.2.15 cell suspension of 2 × 10⁵ cells/mL. 30 µL of the dsRNA (1,000 nM) and 3 µL of Lipofectamine RNAiMAX (manufactured by Thermo Fisher Scientific, Inc.) were diluted with 67 µL of serum-free medium (manufactured by Thermo Fisher Scientific, Inc.) and incubated at room temperature for 5 minutes. 100 µL of this dsRNA-Lipofectamine RNAiMAX mixture was diluted with 400 µL of a cell culture medium to adjust the volume to 500 µL. This diluted mixture was mixed with 500 µL of the above-described HepG 2.2.15 cell suspension, seeded on a 12-well microtiter plate, and incubated in a 5% CO₂ incubator at 37°C for 3 days. Next, the medium was exchanged at 1,000 µL/well with the above-described cell culture medium, and incubation was further carried out at 37°C for 3 days in a 5% CO₂ incubator. Then, the culture supernatant was discarded, the cells were washed with phosphate buffered saline (1,000 µL/well, manufactured by FUJIFILM Wako Pure Chemical Corporation), and total RNA was extracted using an RNeasy Mini Kit (manufactured by Qiagen). 1 µg of the extracted total RNA was reverse transcribed using a PrimeScript RT Reagent Kit with gDNA Eraser (manufactured by Takara Bio Inc.) to prepare cDNA. YTHDC1 and glyceraldehyde-3-phosphate dehydrogenase (GAPDH) as a housekeeping gene, in the prepared cDNA, were quantified by a real-time PCR method, the YTHDC1 expression level corrected by GAPDH was calculated, and then the relative value (%) of the expression level of YTHDC1 in each of the dsRNA-added wells to the expression level of YTHDC1 in the negative control dsRNA-added well was calculated (Fig. 8).

It has been revealed that in a case where the expression level of YTHDC1 is reduced by dsRNAs 3 and 4, the amount of the HBs antigen is reduced whereas the cell viability is not affected. It has also been revealed that the HBV DNA also decreased.

It has been revealed that excellent anti-HBV effects such as inhibition of HBV protein production and reduction of HBV DNA expression level can be obtained by the inhibition of YTHDC1.

In RNAi experiments using dsRNA and the like, there are known cases where a desired effect is exhibited even though the target molecule is not inhibited (the off-target effect). For this reason, in order to correctly interpret results obtained in the RNAi experiment, it is important to show that the obtained result is not due to the off-target effect. In general, in order to deny that the action of a dsRNA is the off-target effect, it is said to effective to confirm that the same action is exhibited by using two or more kinds of dsRNA for one target gene (Nat Cell Biol, Vol. 5, pp. 489 to 490, 2003). In Test Examples of the present invention, it has been revealed that the anti-HBV effects can be obtained by four different kinds of dsRNA. Accordingly, from the results of the above Test Examples, it has been revealed that the pharmaceutical composition that inhibits the expression of the protein belonging to the YTHDC family has the ability to inhibit the production of the HBV protein.

The pharmaceutical composition according to the embodiment of the present invention exhibits an excellent anti-HBV activity and is useful as an anti-hepatitis B virus agent.

## Claims

1. A pharmaceutical composition for use in a method of treating hepatitis B in a subject, the pharmaceutical composition comprising an inhibitor to an expression or a function of a protein belonging to a YTHDC family, wherein the protein belonging to the YTHDC family is YTHDC1.

2. The pharmaceutical composition for use according to claim 1,
wherein the inhibitor to the expression or the function of the protein belonging to the YTHDC family is selected from the group consisting of an antibody, a peptide, a protein, a non-peptide compound, a synthetic compound, an antisense nucleic acid, a double-stranded RNA, and a CRISPR-Cas vector system, each of which has an effective amount.

3. The pharmaceutical composition for use according to claim 2,
wherein the double-stranded RNA is an RNA interference agent.

4. The pharmaceutical composition for use according to claim 1,
wherein the pharmaceutical composition inhibits a production of a hepatitis B virus protein, optionally wherein the hepatitis B virus protein is an HBs antigen protein.

5. The pharmaceutical composition for use according to claim 4,
wherein the pharmaceutical composition reduces an expression level of the hepatitis B virus DNA.

6. The pharmaceutical composition for use according to claim 4 or 5,
wherein the pharmaceutical composition reduces an expression level of a completely closed double-stranded DNA.

7. An in vitro method of screening a substance that inhibits a production of a hepatitis B virus protein, the method comprising:
(a) a step of bringing cells infected with hepatitis B virus or cells expressing hepatitis B virus into contact with a test substance that reduces an expression level of a protein belonging to a YTHDC family or with a test substance that inhibits a function or an activity of the protein; wherein the protein belonging to the YTHDC family is YTHDC1;
(b) a step of measuring an ability of producing a hepatitis B virus protein in the cells infected with hepatitis B virus or the cells expressing hepatitis B virus; and
(c) a step of selecting a substance having an activity of inhibiting a production of the hepatitis B virus protein of the step (b).

## Patentansprüche

1. Pharmazeutische Zusammensetzung zur Verwendung bei einem Verfahren des Behandelns von Hepatitis B bei einer Untersuchungsperson, wobei die pharmazeutische Zusammensetzung einen Inhibitor einer Expression oder einer Funktion eines Proteins, das zu einer YTHDC-Familie gehört, umfasst, wobei das Protein, das zu der YTHDC-Familie gehört, YTHDC1 ist.

2. Pharmazeutische Zusammensetzung zur Verwendung nach Anspruch 1,
wobei der Inhibitor der Expression oder der Funktion des Proteins, das zu der YTHDC-Familie gehört, aus der Gruppe, die aus einem Antikörper, einem Peptid, einem Protein, einer nichtpeptidischen Verbindung, einer synthetischen Verbindung, einer Antisense-Nukleinsäure, einer doppelsträngigen RNA und einem CRISPR-Cas-Vektorsystem besteht, ausgewählt ist, wobei jedes davon eine wirksame Menge aufweist.

3. Pharmazeutische Zusammensetzung zur Verwendung nach Anspruch 2,
wobei die doppelsträngige RNA ein RNA-Interferenzmittel ist.

4. Pharmazeutische Zusammensetzung zur Verwendung nach Anspruch 1,
wobei die pharmazeutische Zusammensetzung eine Produktion eines Hepatitis-B-Virus-Proteins hemmt, wobei optional das Hepatitis-B-Virus-Protein ein HBs-Antigen-Protein ist.

5. Pharmazeutische Zusammensetzung zur Verwendung nach Anspruch 4,
wobei die pharmazeutische Zusammensetzung ein Expressionsniveau der Hepatitis-B-Virus-DNA reduziert.

6. Pharmazeutische Zusammensetzung zur Verwendung nach Anspruch 4 oder 5,
wobei die pharmazeutische Zusammensetzung ein Expressionsniveau einer vollständig geschlossenen doppelsträngigen DNA reduziert.

7. In-vitro-Verfahren zum Screening einer Substanz, die eine Produktion eines Hepatitis-B-Virus-Proteins hemmt, wobei das Verfahren umfasst:
(a) einen Schritt des Inkontaktbringens von mit Hepatitis-B-Virus infizierten Zellen oder von Zellen, die Hepatitis-B-Virus exprimieren, mit einer Testsubstanz, die ein Expressionsniveau eines Proteins, das zu einer YTHDC-Familie gehört, reduziert, oder mit einer Testsubstanz, die eine Funktion oder eine Aktivität des Proteins hemmt, wobei das Protein, das zu der YTHDC-Familie gehört, YTHDC1 ist;
(b) einen Schritt des Messens einer Fähigkeit des Produzierens eines Hepatitis-B-Virus-Proteins in den mit Hepatitis-B-Virus infizierten Zellen oder den Zellen, die Hepatitis-B-Virus exprimieren; und
(c) einen Schritt des Auswählens einer Substanz, die eine Aktivität des Hemmens einer Produktion des Hepatitis-B-Virus-Proteins des Schritts (b) aufweist.

## Revendications

1. Composition pharmaceutique destinée à être utilisée dans une méthode de traitement de l'hépatite B chez un sujet, la composition pharmaceutique comprenant un inhibiteur de l'expression ou de la fonction d'une protéine appartenant à une famille YTHDC, dans laquelle la protéine appartenant à la famille YTHDC est YTHDC1.

2. Composition pharmaceutique destinée à être utilisée selon la revendication 1,
dans laquelle l'inhibiteur de l'expression ou de la fonction de la protéine appartenant à la famille YTHDC est sélectionné dans le groupe constitué d'un anticorps, d'un peptide, d'une protéine, d'un composé non peptidique, d'un composé de synthèse, d'un acide nucléique antisens, d'un ARN bicaténaire et d'un système vectoriel CRISPR-Cas, chacun d'eux ayant une quantité efficace.

3. Composition pharmaceutique destinée à être utilisée selon la revendication 2,
dans laquelle l'ARN bicaténaire est un agent d'interférence ARN.

4. Composition pharmaceutique destinée à être utilisée selon la revendication 1,
dans laquelle la composition pharmaceutique inhibe une production d'une protéine du virus de l'hépatite B, éventuellement dans laquelle la protéine du virus de l'hépatite B est une protéine antigène HBs.

5. Composition pharmaceutique destinée à être utilisée selon la revendication 4,
dans laquelle la composition pharmaceutique réduit un niveau d'expression de l'ADN du virus de l'hépatite B.

6. Composition pharmaceutique destinée à être utilisée selon la revendication 4 ou la revendication 5,
dans laquelle la composition pharmaceutique réduit un niveau d'expression d'un ADN bicaténaire complètement fermé.

7. Méthode in vitro de criblage d'une substance qui inhibe une production d'une protéine du virus de l'hépatite B, la méthode comprenant :
(a) une étape consistant à mettre des cellules infectées par le virus de l'hépatite B ou des cellules exprimant le virus de l'hépatite B en contact avec une substance d'essai qui réduit un niveau d'expression d'une protéine appartenant à une famille YTHDC ou avec une substance d'essai qui inhibe une fonction ou une activité de la protéine ; dans laquelle la protéine appartenant à la famille YTHDC est YTHDC1 ;
(b) une étape de mesure d'une capacité de production d'une protéine du virus de l'hépatite B dans les cellules infectées par le virus de l'hépatite B ou les cellules exprimant le virus de l'hépatite B ; et
(c) une étape de sélection d'une substance ayant une activité d'inhibition d'une production de la protéine du virus de l'hépatite B de l'étape (b).
